# EUROPÄISCHE PATENTSCHRIFT

(11) **EP 0 998 316 B1**
(45) Veröffentlichungstag und Bekanntmachung des Hinweises auf die Patenterteilung: **03.11.2004**
(21) Anmeldenummer: 98931877.9
(22) Anmeldetag: 17.07.1998
(51) Int. Cl.: A61M 5/315

(54) **DOSIERKNOPFSICHERUNG AN EINER VORRICHTUNG ZUR DOSIERTEN VERABREICHUNG EINES INJIZIERBAREN PRODUKTS**
SYSTEM FOR LOCKING A DOSING BUTTON IN A DEVICE FOR THE ADMINISTRATION OF A PRODUCT TO BE INJECTED
SYSTEME DE BLOCAGE POUR BOUTON DE DOSAGE D'UN DISPOSITIF SERVANT A ADMINISTRER UN PRODUIT INJECTABLE

(30) Priorität: 18.07.1997 DE 19730999
(43) Veröffentlichungstag der Anmeldung: 10.05.2000
(73) Patentinhaber: TecPharma Licensing AG, 3401 Burgdorf (CH)
(72) Erfinder: KIRCHHOFER, Fritz, CH-3454 Sumiswald (CH); HERTIG, Guido, CH-3322 Mattstetten (CH)
(74) Vertreter: Wess, Wolfgang, Dipl.-Ing.
(86) Internationale Anmeldenummer: PCT/CH1998/000313
(87) Internationale Veröffentlichungsnummer: WO 1999/003522

(56) Entgegenhaltungen:
- WO-A-88/08725
- WO-A-92/18179
- WO-A-97/17096

## Beschreibung

Die Erfindung betrifft eine Vorrichtung zur dosierten Verabreichung eines injizierbaren Produkts nach mit einer Dosierknopfsicherung dem Oberbegriff von Anspruch 1.

Vorrichtungen zur dosierten Verabreichung eines injizierbaren Produkts sind insbesondere in der Insulinbehandlung bekannt. Dabei handelt es sich beispielsweise um sogenannte Injektionspens, mit denen ein Diabetiker sich eine gewünschte Dosis Insulin selbst injizieren kann. Solche Injektionspens weisen einen sogenannten Dosierknopf auf, der wie der Betätigungsknopf eines Kugelschreibers aus einer Gehäuseöffnung des Injektionspens herausragt. Zum Vorbereiten der Injektion wird die zu injizierende Dosis durch Drehen des Dosierknopfs relativ zum Gehäuse ausgewählt bzw. eingestellt. Durch das Drehen des Dosierknopfs wird ein Antriebsmittel voreingestellt. Zum Injizieren wird der Dosierknopf dann aus einer ersten Endposition ein Stück weit in das Gehäuse bis zu einer zweiten Endposition hineingedrückt und dabei das voreingestellte Antriebsmittel betätigt. Dieses wiederum wirkt in der voreingestellten Weise auf ein Fördermittel, im allgemeinen ein Kolben, der in einer mit Insulin gefüllten Ampulle verschiebbar aufgenommen ist, und unter der Einwirkung des Antriebsmittels die voreingestellte Insulindosis aus der Ampulle durch eine Injektionsnadel hindurch verdrängt. Der Dosierknopf wird am Ende der Injektion in seiner zweiten Endposition verriegelt, aus der er sich erst wieder unmittelbar vor der nächsten Injektion in seine erste Endposition zurück bewegt, in der ein erneutes Dosieren wieder möglich ist.

Bei solch einer, beispielsweise aus der EP 0 730 876 A2 und der DE 41 12 259 A1 bekannten Vorrichtung ist ein Verschieben des Dosierknopfs aus seiner verriegelten zweiten Endposition in die erste Endposition nur möglich, wenn eine Anzeigeeinrichtung die Nullstellung des Dosierknopfs anzeigt. Die Ausgangsstellung des Dosierknopfs vor Dosierung der mit der nächsten Injektion zu verabreichenden Produktmenge ist definiert und mit der Anzeige abgestimmt. Allerdings ist der hierfür zu betreibende konstruktive Aufwand erheblich.

Die Erfindung hat es sich zur Aufgabe gemacht, mit möglichst einfachen Mitteln zu gewährleisten, dass eine angezeigte Drehstellung eines Dosierknopfs einer Vorrichtung zur dosierten Verabreichung eines injizierbaren Produkts mit der tatsächlichen Drehstellung des Dosierknopfs beim Dosieren übereinstimmt.

Diese Aufgabe wird durch den Gegenstand von Anspruch 1 gelöst.

Eine Vorrichtung zur dosierten Verabreichung eines injizierbaren Produkts weist ein Gehäuse mit einer Aufnahme für das Produkt auf. Eine auswählbare Dosis des in einem Behältnis enthaltenen Produkts wird für eine Injektion mittels eines Fördermittels aus dem Behältnis gefördert. Das Behältnis kann unmittelbar durch das Gehäuse, d.h. durch die Aufnahme des Gehäuses gebildet werden. Vorzugsweise dient als Behältnis eine Ampulle, die in der als Ampullenfach ausgebildeten Aufnahme des Gehäuses aufgenommen ist. Das Fördermittel wird bevorzugterweise durch einen im Behältnis verschiebbar aufgenommenen Kolben gebildet. Das Fördermittel kann beispielsweise aber auch durch einen Quetschmechanismus, bei dem ein nachgiebiger Schlauch zwischen zwei Quetschstellen gequetscht wird, d.h. eine Peristaltikpumpe, gebildet werden. Grundsätzlich wären auch dosierbare Rotorpumpen einsetzbar. Bei dem injizierbaren Produkt handelt es sich in erster Linie um eine flüssige Wirkstofflösung, beispielsweise Insulin oder andere Medikamentflüssigkeiten.

Ein Antriebsmittel für das Fördermittel wird bei Verwendung eines Kolbens vorzugsweise durch eine Kolbenstange gebildet, die gegen den Kolben drückend den Kolben in Richtung auf einen Auslass des Behältnisses bewegt, so dass das Produkt aus dem Behältnis verdrängt wird.

Mit dem Antriebsmittel gekoppelt ist ein im Gehäuse verschiebbar gelagerter Dosierknopf, der bei einem Verschieben aus einer ersten Verschiebeposition in eine zweite Verschiebeposition das Antriebsmittel derart betätigt, dass die gewählte Produktdosis vom Fördermittel gefördert wird. Die Dosis wird in der ersten Position des Dosierknopfs durch Verdrehen des Dosierknopfs aus einer ersten Drehstellung für Nullförderung in eine der gewählten Dosis zugeordnete zweite Drehstellung gewählt. Die Kopplung ist vorzugsweise eine mechanische; grundsätzlich kann aber eine andere Kopplung, beispielsweise eine elektrische oder eine elektrisch/mechanische, zum Einsatz kommen.

Zum Anzeigen der Drehstellung des Dosierknopfs und damit der gewählten Produktdosis sind eine gehäusefeste erste Anzeigenhülse und eine mit dem Dosierknopf drehbare zweite Anzeigenhülse vorgesehen. Die Anzeigenhülsen sind mit Anzeigemitteln versehen, die ein Ablesen der gewählten Dosis aufgrund der Drehstellung der Anzeigenhülsen zueinander gestatten. Vorzugsweise umgibt eine der Anzeigehülsen die andere. In dieser Anordnung sind auf einer Skala der inneren Anzeigenhülse Dosiswerte oder Dosiswerte repräsentierende Zeichen aufgetragen, die durch ein Fenster in der äusseren Anzeigenhülse ablesbar sind. Die Anzeigenhülsen könnten jedoch auch entlang einer gemeinsamen Längsachse hintereinander angeordnet sein, wobei die relative Drehlage dann beispielsweise mittels einer Markierung auf der Mantelfläche der einen Anzeigenhülse, die einer entsprechenden Skala auf der anderen Anzeigenhülse gegenüberläge, angezeigt würde. Vorzugsweise ist die innere Anzeigenhülse gehäusefest angeordnet oder wird selbst durch einen Teil des Gehäuses gebildet, und die äussere Anzeigenhülse wird durch Drehen des Dosierknopfs um die innere Anzeigenhülse gedreht. Grundsätzlich könnte in dieser schalenartigen Anordnung der Anzeigenhülse auch die innere mit dem Dosierknopf drehbar und die äussere gehäusefest sein.

Um vor einer Injektion einen definierten Ausgangszustand der Vorrichtung zu gewährleisten, ist eine Dosierknopfsicherung in Form einer Verschiebesicherung vorgesehen. Diese Dosierknopfsicherung verhindert ein unkontrolliertes, d.h. unbeabsichtigtes Verschieben des Dosierknopfs aus der zweiten Position zurück in die erste Position. Der Dosierknopf kann sich in seine erste Position nur zurückbewegen, wenn die Anzeigenhülsen in einer Nullstellung zueinander stehen, in der sie die Dosis "Null" anzeigen. In der gesicherten zweiten Position ist der Dosierknopf vorzugsweise nicht nur gegen Verschieben, sondern auch gegen Verdrehen relativ zum Gehäuse gesichert. Die Dosierknopfsicherung kann als Verschiebe- und gleichzeitig Verdrehsicherung ausgebildet sein. Eine Verdrehsicherung kann auch anderweitig bereitgestellt werden. Grundsätzlich wäre jedoch auch denkbar, dass der Dosierknopf in seiner vorderen, zweiten Position leer dreht, indem er von dieser Position vom Antriebsmittel entkoppelt ist.

Erfindungsgemäss wird die Dosierknopfsicherung durch einfaches Drücken eines Startknopfs gelöst, der jedoch von der mit dem Dosierknopf drehbaren zweiten Anzeigenhülse für einen äusseren Zugriff, nämlich zum Drücken, nur in der Nullstellung der ersten und zweiten Anzeigenhülse freigegeben wird.

Die erfindungsgemässe Entsicherung der Dosierknopfsicherung baut einfach und kompakt und ist nicht zuletzt deshalb besonders robust und betriebssicher, indem zum Abschirmen und Freigeben des Startknopfs eine Hülse verwendet wird, die in der hinteren ersten Dosierknopfposition gleichzeitig mit dem Dosierknopf verdrehbar ist, wobei die Freigabe des Startknopfs in Abhängigkeit von der Drehstellung dieser Hülse erfolgt.

Nach der Erfindung weist die zweite Anzeigenhülse eine Durchbrechung auf, die in der Nullstellung von erster und zweiter Anzeigenhülse über dem Startknopf liegt, so dass der Startknopf zum Eindrücken freigegeben ist. Solange die zweite Anzeigenhülse sich jedoch nicht in der Nullstellung zur ersten Anzeigenhülse befindet, überdeckt sie den Dosierknopf, so dass dieser vor äusserem Zugriff abgeschirmt ist und daher ein Entsichern der Verschiebesicherung verhindert wird. Die Durchbrechung ist in einem ersten Ausführungsbeispiel ein einfacher offener Durchbruch, der ausreichend gross ist, so dass ein Benutzer in den Durchbruch eingreifen kann. In einem zweiten Ausführungsbeispiel wird durch die Durchbrechung an der zweiten Anzeigenhülse eine Eindrückhilfe, vorzugsweise in Form einer Lasche, geschaffen, die radial nach innen gedrückt werden kann und elastisch zurückfedert, wenn der Betätigungsdruck von ihr genommen wird.

Die Erfindung findet bevorzugt Verwendung bei Injektionsgeräten, insbesondere in der Bauform eines Pen; sie ist auch bei Infusions/Injektions-Vorrichtungen verwendbar.

Bevorzugte Ausführungsbeispiele der Erfindung werden nachfolgend anhand von Figuren erläutert. Es zeigen:
- Fig. 1: einen Injektionspen mit einer Dosierknopfsicherung, wobei Mittel zum Lösen der Sicherung und Dosisanzeigemittel nach einem ersten Ausführungsbeispiel ausgebildet sind;
- Fig. 2: den Längsschnitt nach Fig. 1;
- Fig. 3: eine Anzeigenhülse nach den Figuren 1 und 2;
- Fig. 4: den Querschnitt A-A nach Fig. 2; und
- Fig. 5: einen Injektionspen, mit einer Dosierknopfsicherung, wobei Mittel zum Lösen der Sicherung und Dosisanzeigemittel nach einem zweiten Ausführungsbeispiel ausgebildet sind.

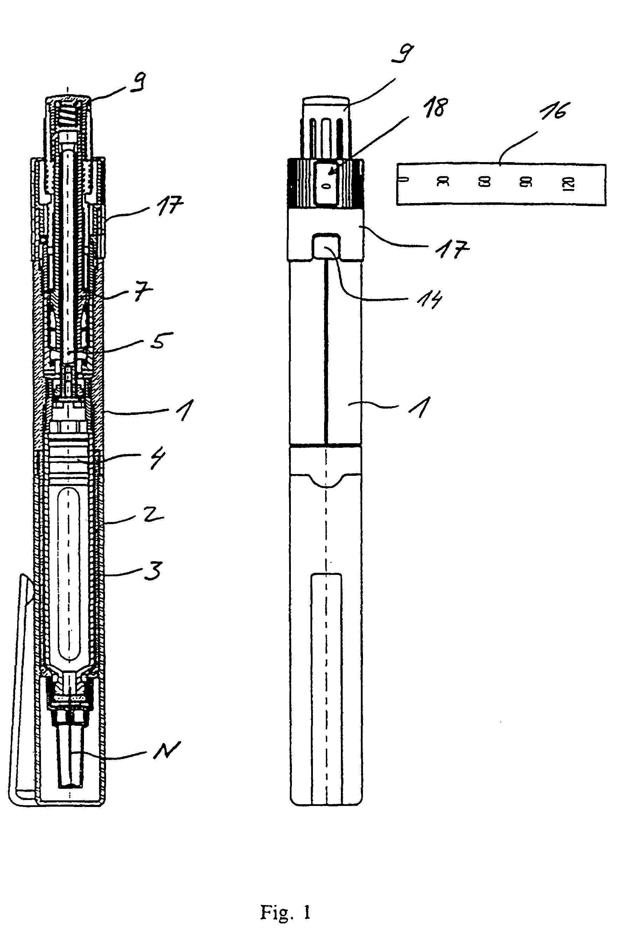

In Fig. 1 ist ein Injektionsgerät in Form eines sogenannten Injektionspen dargestellt. Injektionspens dienen in erster Linie zur mehrfachen, jeweils dosierten Selbstinjektion einer flüssigen Wirkstofflösung, beispielsweise Insulin oder Hormonpräparate.

Das Injektionsgerät weist ein langgestrecktes Gehäuse mit einem vorderen Gehäuseteil, das als Aufnahme 2 für die zu verabreichende Wirkstofflösung dient, und ein hinteres Gehäuseteil 1 auf. Im Gehäuseteil 1 ist ein Antrieb untergebracht, der ein Fördermittel, im Ausführungsbeispiel ein Kolben 4, antreibt. In der Aufnahme 2 ist eine mit der Wirkstofflösung gefüllte Ampulle 3 aufgenommen. Die Wirkstofflösung wird durch Vorschub des Kolbens 4 auf einen Ampullenauslass zu durch eine sich an den Ampullenauslass anschliessende Injektionsnadel N verdrängt.

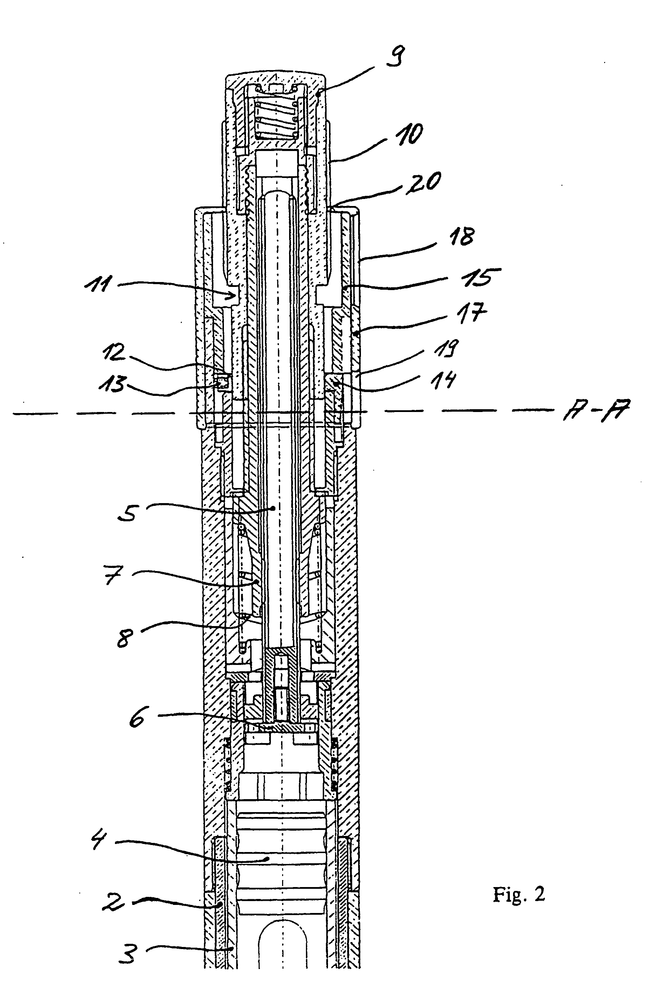

Der Vorschub des Kolbens 4 wird durch Andruck einer Gewindestange 5 bewirkt. Die Gewindestange 5 bildet das Abtriebsglied eines Spindeltriebs, der im Ausführungsbeispiel mit der Gewindestange 5 und einer sie umgebenden Gewindehülse 7 als Antriebsglied einstufig ausgebildet ist. Zum Vorschub des Kolbens 4 wird die Gewindehülse 7 zusammen mit der Gewindestange 5 durch Betätigen eines Dosierknopfs 9 ihrerseits in Kolbenvorschubrichtung auf die Rückseite des Kolbens 4 zu gegen eine elastische Rückstellkraft vorgeschoben. Der Dosierknopf 9, die Antriebsmittel 5, 7 und der Kolben 4 werden entlang einer gemeinsamen, in Fig. 1 punktstrichliert eingezeichneten Achse, der Verschiebeachse, geradverschoben. Dabei ist die Weglänge, um die der Dosierknopf 9 und die Antriebsmittel 5, 7 bei einer Betätigung bzw. Injektion verschoben werden, stets gleich. Der Vorschub erfolgt aus einer hinteren ersten Position, die in den Figuren 1 und 2 dargestellt ist, in eine vordere zweite Position, die der Dosierknopf 9 und im Ausführungsbeispiel mit ihm auch die Antriebsmittel 5, 7 nach erfolgter Injektion einnehmen. Variierbar ist hierbei die vom Kolben 4 unter der Einwirkung des Antriebs 5, 7 zurückgelegt Weglänge und damit die pro Injektion verabreichte Wirkstoffdosis.

Die mittels des Dosierknopfs 9 gewählte Dosis wird in einem Fenster 18 einer Anzeigenhülse 17 angezeigt. Die kontinuierlich, oder wie im Ausführungsbeispiel diskret einstellbaren Dosen sind auf einer gehäusefesten Ringskala 16 aufgetragen, die von der Anzeigenhülse 17 im Bereich ihres Fensters 18 konzentrisch umgeben wird. Ein gehäusefest angeordnetes Hülsenbauteil, auf dem die Ringskala aufgebracht ist, wird im folgenden als erste Anzeigenhülse und die mit dem Dosierknopf 9 drehbare Anzeigenhülse 17 als zweite Anzeigenhülse bezeichnet. Erfindungsgemäss wirkt die mit dem Dosierknopf 9 drehbare zweite Anzeigenhülse 17 mit einem Startknopf 14 derart zusammen, dass die Drehstellung des Dosierknopfs 9 in der dargestellten hinteren ersten Position des Dosierknopf 9 mit der im Fenster 18 ablesbaren Dosis in stets definierter Weise korreliert ist.

In Fig. 2 ist der hintere Teil des Längsschnitts nach Fig. 1 vergrössert dargestellt. Der Dosierknopf 9 befindet sich bezüglich seines Verschiebewegs in seiner hinteren ersten Position, wie in Fig. 1. In der ersten Position wird die mit der nächsten Injektion zu verabreichende Wirkstoffdosis durch Drehen des Dosierknopfs 9 um seine Längsachse vorgewählt, d.h. es erfolgt die Dosierung.

Der Dosierknopf 9 weist ein an seinem hinteren Ende durch ein Einsatzstück verschlossenes Hülsenteil auf. Das Hülsenteil des Dosierknopfs 9 durchragt eine hintere Stirnfläche des Gehäuses 1. Im Bereich des Hülsenteils ist der Dosierknopf 9 verdrehgesichert mit der Gewindehülse 7 verbunden. Durch Drehen des Dosierknopfs wird folglich die Gewindehülse 7 um ihre Mittelachse zwangsweise mit verdreht. Die Gewindestange 5 ist im Gehäuse 1 verdrehsicher geradgeführt, so dass ein Verdrehen der Gewindehülse 7 zwangsweise eine Geradverschiebung der Gewindestange 5 bewirkt. An ihrem dem Kolben 4 zugewandten vorderen Ende weist die Gewindestange 5 einen Flansch oder Stempel 6 auf, mit dem sie bei Betätigung des Dosierknopfs, d.h. bei dessen Verschieben aus der dargestellten hinteren ersten Position in eine vordere zweite Position gegen den Kolben 4 drückend diesen Kolben 4 in der Ampulle 3 vorschiebt. Die Dosierung erfolgt in der gerade geschilderten Weise durch das Einstellen des lichten Abstands zwischen der vorderen Stirnfläche des Gewindenstangenstempels 6 und der rückwärtigen Stirnfläche des Kolbens 4 in der hinteren ersten Position des Dosierknopfs 9. Der Verschiebeweg des Gewindestangenstempels 6 ist bei jeder Injektion gleich lang. Das Vorschieben erfolgt gegen die elastische Rückstellkraft einer Druckfeder 8, die zwischen einem Ansatz des Gehäuses 1 und einem entsprechenden Gegenansatz an der Gewindehülse 7 angeordnet ist. Die Druckfeder 8 ist bestrebt, die gesamte, im wesentlichen aus der Gewindehülse 7, der Gewindestange 5 und dem Dosierknopf 9 bestehende Betätigungseinrichtung in ihre hintere erste Position zurückzudrücken.

Um unkontrollierte Injektionen zu verhindern, die nie auszuschliessen wären, falls die Betätigungseinrichtung nach jeder Injektion von selbst wieder in ihre Ausgangsposition, d.h. in ihre hintere erste Position zurückfedern würde, ist eine Verschiebesicherung vorgesehen. Die Verschiebesicherung weist einen den Hülsenteil des Dosierknopfs 9 umgreifenden Ring mit einem Nocken 12 auf, der in eine Ausnehmung 11 in einer äusseren Mantelfläche des Hülsenteils des Dosierknopfs 9 eingreift, wenn der Dosierknopf 9 sich in seiner vorderen zweiten Position befindet. Die Ausnehmung 11 ist im Ausführungsbeispiel als umlaufende Ringnut ausgebildet.

Damit der Nocken 12 in die Ausnehmung 11 einschnappt, wenn sich der Dosierknopf 9 in seiner vorderen zweiten Position befindet, ist der Nocken 12 durch eine Druckfeder 13, die zwischen dem Gehäuseteil 1 und der Nockenrückseite angeordnet ist, radial auf die äussere Mantelfläche des Hülsenteils des Dosierknopfs 9 vorgespannt. Der Ring mit dem Nocken 12 weist dem Nocken 12 diametral gegenüberliegend einen Startknopf 14 auf. Wird der Nocken 12 in der vorderen zweiten Stellung des Dosierknopfs 9 in die Ausnehmung 11 hineingedrückt, so wird der Startknopf 14 nach aussen in eine entsprechende Öffnung des Gehäuses 1 hinein- oder auch hindurchgedrückt. Umgekehrt wird in der durch den Nocken 12 gesicherten zweiten Position des Dosierknopfs 9 der Nocken 12 entgegen der elastischen Rückstellkraft der Druckfeder 13 durch manuell auf den Startknopf 14 ausgeübten Druck aus der Ausnehmung 11 herausbewegt und die Verschiebesicherung dadurch gelöst. Der so entsicherte Dosierknopf 9 wird durch die elastische Rückstellkraft der Druckfeder 8 mitsamt der Gewindehülse 7 und der Gewindestange 5 wieder in seine hintere, erste Position zurückgedrückt.

Die zweite Anzeigenhülse 17 wird in der hinteren ersten Position des Dosierknopfs 9 zwangsweise zusammen mit dem Dosierknopf 9 verdreht. Sie ist mit dem Dosierknopf 9 formschlüssig verbunden. Im Ausführungsbeispiel dreht sie mit dem Dosierknopf 1 : 1 mit; grundsätzlich wäre auch eine Über- oder Untersetzung der Drehbewegung des Dosierknopfs 9 auf die zweite Anzeigenhülse 17 denkbar. Die formschlüssige Verdrehsicherung wird durch zahnartigen Eingriff zwischen dem Dosierknopf 9 und der zweiten Anzeigenhülse 17 bewirkt. Hierfür weist das zylindrische, im Ausführungsbeispiel kreiszylindrische, Hülsenteil des Dosierknopfs 9 über den Umfang einer äusseren Mantelfläche entsprechend der Skalenteilung gleichmässig verteilt angeordnete, sich axial erstreckende Erhebungen und Vertiefungen 10 auf, in die an einer inneren Mantelfläche der zweiten Anzeigenhülse 17 entsprechend angeordnete Erhebungen und Vertiefungen 20 eingreifen und ein Verdrehen der zweiten Anzeigenhülse 17 gegenüber dem Dosier- knopf 9 verhindern.

Die zweite Anzeigenhülse 17 umgibt die erste Anzeigenhülse 15 und diese wiederum den Dosierknopf 9. Im Ausführungsbeispiel sind das Hülsenteil des Dosierknopfs 9 und die beiden Anzeigenhülsen 15 und 17 konzentrisch angeordnet. Es wäre auch möglich, die mit dem Dosierknopf 9 drehende Anzeigenhülse innerhalb der gehäusefesten Anzeigenhülse anzuordnen, und ebenso wäre es in Abwandlung des Ausführungsbeispiels möglich, die beiden Anzeigenhülsen hintereinander anzuordnen.

Die zweite Anzeigenhülse 17 ist am Gehäuse 1 in ihrer axialen Position fixiert und um ihre Längsachse drehgelagert. Im Ausführungsbeispiel fallen die Verschiebeachse des Dosierknopfs, aber auch die der Betätigungseinrichtung 5, 7, 9 und die Drehachse der zweiten Anzeigenhülse 17 zusammen.

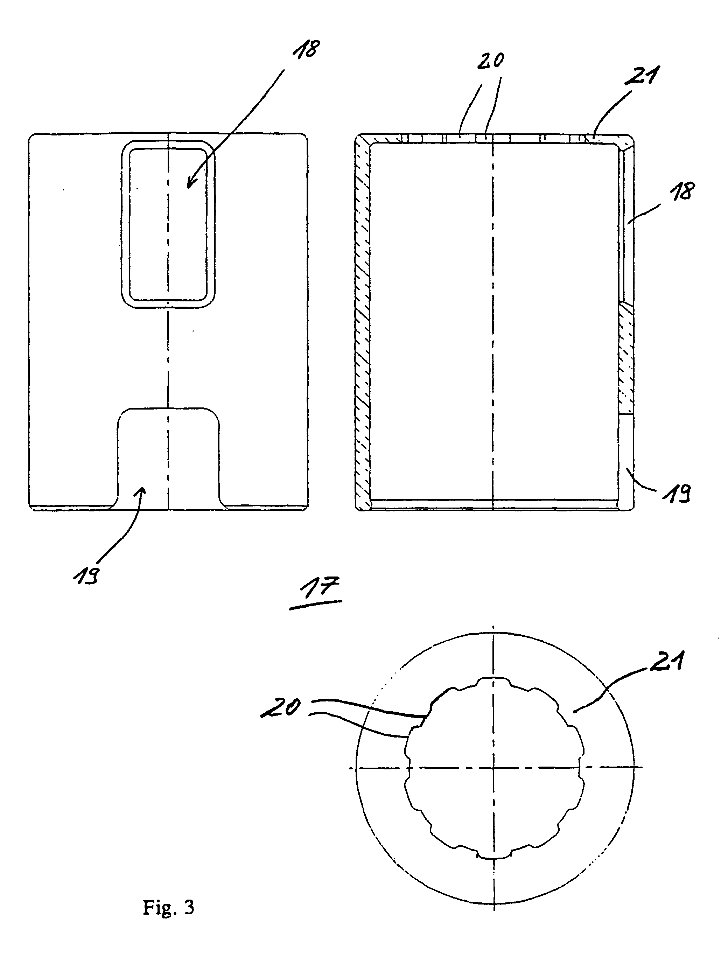

Figur 3 zeigt die zweite Anzeigenhülse 17 in einer Ansicht, einem Längsschnitt und einer Draufsicht auf die hintere Stirnfläche mit den zahnartigen Erhebungen und Vertiefungen 20. Die zweite Anzeigenhülse 17 ist mit Ausnahme ihres Anzeigenfensters 18 und einer Durchbrechung 19 als geschlossene Hülle ausgebildet. Im Ausführungsbeispiel ist sie einfach kreiszylindrisch mit einer umlaufenden, radial nach innen abkragenden Schulter. Die Erhebungen und Vertiefungen 20 sind umlaufend an einem inneren Umfangsrand der Schulter ausgebildet. Mit dieser Schulter überkragt die zweite Anzeigenhülse 17 den stirnseitigen hinteren Umfangsrand des Gehäuses 1, das in diesem hinteren Bereich durch die erste Anzeigenhülse 15 gebildet wird (Fig. 2). In ihrem hinteren Mantelbereich, in dem das Fenster 18 ausgelassen ist, ist die zweite Anzeigenhülse 17 der besseren Greifbarkeit wegen gerauht. In ihrem vorderen Mantelbereich weist sie die Durchbrechung 19 auf, deren Abmessung so gewählt ist, dass der Startknopf 14 durch diese Durchbrechung 19 hindurch vom Verwender des Injektionsgeräts bedient, d.h. nach innen auf die Verschiebeachse zu gedrückt werden kann. Um die Fläche der Durchbrechung 19 möglichst klein halten zu können, ist die Durchbrechung 19 als ein zum vorderen Umfangsrand der zweiten Anzeigenhülse 17 sich öffnender Einschnitt ausgebildet, in den der Verwender vom Rand her eingreifen kann. Das Anzeigenfenster 18 und die Durchbrechung 19 sind in Längsrichtung der zweiten Anzeigenhülse 17 nebeneinander angeordnet, so dass der Startknopf 14 für den Verwender dann sichtbar wird bzw. die Durchbrechung 19 über dem Startknopf 14 zu liegen kommt, wenn im Anzei- gefenster 18 die Nulldosis angezeigt wird.

Der Formschluss zwischen der zweiten Anzeigenhülse 17 und dem Dosierknopf 9 besteht lediglich bezüglich der Drehbewegung, während das Verschieben des Dosierknopfs 9 relativ zur zweiten Anzeigenhülse 17 hierdurch nicht behindert wird. Die Erhebungen und Vertiefungen 10 am Dosierknopf 9 erstrecken sich am Dosierknopf 9 bzw. dessen Hülsenteil auch nur so weit in Richtung auf das hintere Ende des Dosierknopfs 9 zu, dass der formschlüssige Eingriff der zweiten Anzeigenhülse 17 in der vorderen zweiten Position des Dosierknopfs 9 nicht besteht. In der zweiten Dosierknopfposition ist die zweite Anzeigenhülse 17 um ihre Drehachse frei drehbar.

Die freie Verdrehbarkeit der Anzeigenhülse 17 wird lediglich der ersten Anzeigenhülse 15 bzw. dem Gehäuse 1 gegenüber eingeschränkt, indem die zweite Anzeigenhülse 17 dazu verwendet wird, die mit dem Dosierknopf 9 wählbare maximale Dosis zu limitieren. Der Dosierknopf 9 kann im Ausführungsbeispiel um maximal 360° bzw. nahezu 360° verdreht werden.

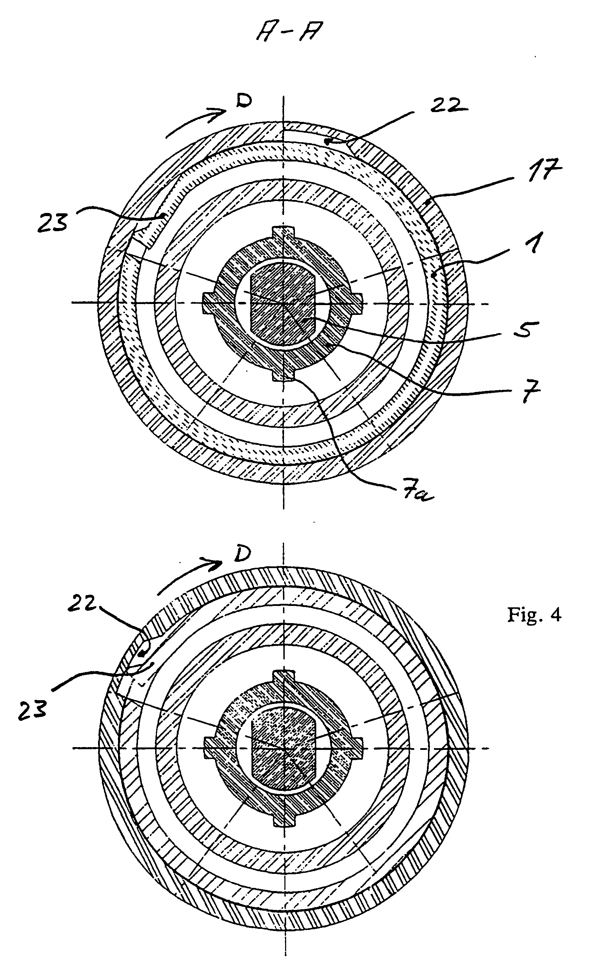

Dies wird, wie in Figur 4 aus den beiden Ansichten des Querschnitts A-A nach Fig. 2 zu erkennen ist, dadurch erreicht, dass die zweite Anzeigenhülse 17 in einer Drehrichtung, in der die im Fenster 18 angezeigten Dosiswerte ansteigen, gegen einen gehäuseseitigen Anschlag geführt wird. Ein Verdrehen des Dosierknopfs 9 in die andere Drehrichtung, d.h. in Richtung abfallender Dosiswerte, wird bereits durch bekannte Rastmittel, beispielsweise eine Ratsche, verhindert, die zwischen dem Dosierknopf 9 oder der Antriebshülse 7 und dem Gehäuse 1 wirken. Die Drehrichtung der zweiten Anzeigenhülse 17 und des Dosierknopfs 9 beim Dosieren ist in den beiden Schnittansichten der Figur 4 mit einem Pfeil D markiert.

Der Anschlag für die Drehbewegung der zweiten Anzeigenhülse 17 wird durch eine Einkerbung 22 und eine mit einer Rastnase bzw. einem Nocken in die Einkerbung 22 eingreifende Federzunge 23 gebildet. Die Einkerbung 22 erstreckt sich axial über eine innere Mantelfläche der zweiten Anzeigenhülse 17. Die federnde Zunge 23 wird zwischen zwei sich in Umfangsrichtung am Gehäuse 1 erstreckenden Schlitzen mit einem diese beiden Schlitze verbindenden kurzen Axialschlitz gebildet, d.h. das Gehäuse 1 ist im Bereich seiner von der zweiten Anzeigenhülse 17 umhüllten Mantelfläche U-förmig geschlitzt. Von dem federnden Ende der Zunge 23 ragt die Rastnase bzw. der Nocken auf der Höhe der Einkerbung 22 radial nach aussen.

Beim Verdrehen der zweiten Anzeigenhülse 17 in Richtung des Pfeils D im Zuge eines Dosiervorgangs kann die zweite Anzeigenhülse 17, und über die mechanische Kopplung der Dosierknopf 9, bis maximal gegen eine bezüglich der Drehrichtung D rückwärtige Anschlagfläche der Einkerbung 22 verdreht werden. Die obere Ansicht der Fig. 4 zeigt die zweite Anzeigenhülse 17 dabei kurz nach Beginn eines Dosiervorgangs und die untere Ansicht kurz vor Ende der Auswahl der Maximaldosis.

In Ausbildung des Anschlags springt die Einkerbung 22 zur einen Seite hin von der inneren Mantelfläche der zweiten Anzeigenhülse 17 radial nach aussen zurück, während die Einkerbung 22 an der gegenüberliegenden Seite zur Radialen schräg verläuft, so dass die zweite Anzeigenhülse 17 über diese schräge Begrenzungsfläche der Einkerbung 22 hinweg dem Gehäuse 1 gegenüber verdreht werden kann. In diese Drehrichtung, d.h. gegen die eingezeichnete Drehrichtung D, wird die zweite Anzeigenhülse 17 nach der Injektion wieder in ihre Nullstellung zurückgedreht. Das Zusammenwirken der Einkerbung 22 mit der federnden Zunge 23 bewirkt auch gleichzeitig über die reine Anschlagfunktion beim Dosieren hinaus, dass die zweite Anzeigenhülse 17 in ihrer Nullstellung arretiert wird, in dem Sinne, dass der Verwender es zumindest fühlt, wenn er die zweite Anzeigenhülse 17 vor der nächsten Injektion in ihre Nullstellung dreht; gleichzeitig wird die Handhabung des Injektionspens erleichtert, da die zweite Anzeigenhülse 17 in ihrer Nullstellung gehalten wird und nicht beim Drücken des Startknopfs 14 durch selbsttätiges Verdrehen stören kann.

Figur 4 zeigt auch die Verdrehsicherung der Gewindestange 5 gegenüber dem Gehäuse 1, nämlich zwei sich gegenüberliegende Abflachungen. Dargestellt ist auch die Verdrehsicherung der Gewindehülse 7 in Form von radial nach aussen stehenden Führungen 7a, die unmittelbar nach dem Drücken des Dosierknopfs 9 in entsprechenden gehäuseseitigen Ausnehmungen geradgeführt werden.

Wird der Dosierknopf 9 und damit im Ausführungsbeispiel die gesamte Betätigungseinrichtung 5, 7, 9 zum Injizieren der gewählten Dosis in die zweite Position bewegt, so gleitet das Hülsenteil des Dosierknopfs 9 am Nocken 12 entlang. Der Startknopf 14 befindet sich dabei in einer in radialer Richtung zur Verschiebeachse gesehen inneren Stellung. Hat der Dosierknopf 9 seine zweite Position erreicht, so befindet sich die Ausnehmung 11 auf der Höhe des Nockens 12. Der Nocken 12 schnappt aufgrund der Kraft der Druckfeder 13 in die Ausnehmung 11 am Dosierknopf 9 ein. Da der Nocken 12 und der Startknopf 14 über einen Ring starr miteinander verbunden sind, wird der Startknopf 14 beim Einschnappen des Nockens 12 radial von der Verschiebeachse weg nach aussen durch eine entsprechende Öffnung im Gehäuse 1 verschoben. Die zweite Anzeigenhülse 17 erstreckt sich jedoch ausgehend von ihrer hinteren Stirnseite so weit über die zweite Anzeigenhülse 15 bzw. das Gehäuse 1, dass sie den Startknopf 14 noch überdeckt und auf diese Weise gegen einen äusseren Zugriff abschirmt. Dabei steht der Startknopf 14 nicht oder nur soweit radial dem Gehäuse 1 bzw. der ersten Anzeigenhülse 15 gegenüber hervor, dass er die Drehbewegung der zweiten Anzeigenhülse 17 nicht behindert. Lediglich in der Nullstellung der zweiten Anzeigenhülse 17, in der ihr Anzeigenfenster 18 über dem der Nulldosis entsprechenden Dosiswert der Ringskala 16 steht, gibt sie den Zugriff auf den Startknopf 14 frei.

In dieser Stellung der zweiten Anzeigenhülse 14 wird durch Drücken des Startknopfs 14 der Nocken 12 gegen die Kraft der Druckfeder 13 aus der Ausnehmung 11 herausgedrückt und die Betätigungseinrichtung 5, 7, 9, insbesondere der Dosierknopf 9, durch die Druckfeder 8 in die hintere erste Position des Dosierknopfs 9 verschoben. Unmittelbar beim Einsetzen der Rückschiebebewegung kommen auch wieder die Erhebungen und Vertiefungen 10 und 20 des Dosierknopfs 9 und der zweiten Anzeigenhülse 17 in formschlüssigen Eingriff. Der Dosierknopf 9 und die zweite Anzeigenhülse 17 sind in diesem Moment sogleich wieder gegeneinander verdrehgesichert. Ein etwaiges geringfügiges Verdrehen der zweiten Anzeigenhülse 17 im Moment des gegenseitigen Eingreifens der Erhebung und Vertiefungen 10 und 20 ist in jedem Fall so gering, dass die feste Zuordnung, der Dosierknopfdrehstellung und der Anzeige, gewährleistet bleibt.

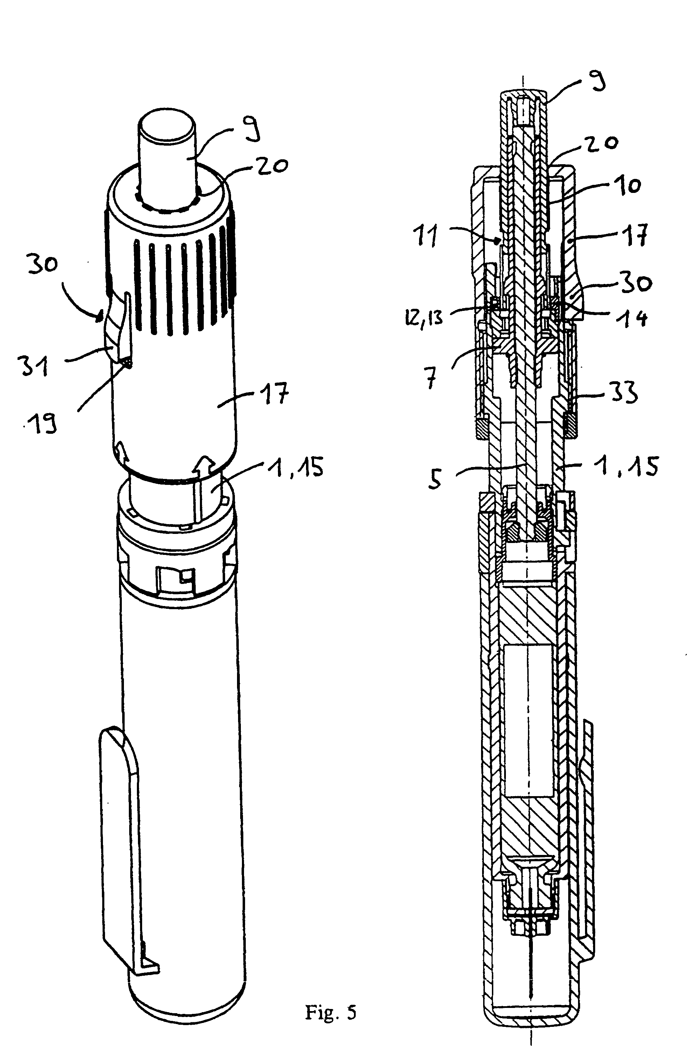

Figur 5 zeigt in einer Ansicht und in einem Längsschnitt einen Injektionspen, der zum Lösen der Dosierknopfsicherung eine alternative Ausbildung der zweiten Anzeigenhülse 17 aufweist. Ferner ist die Skala zum Anzeigen der eingestellten Dosis nicht einfach ringförmig auf der ersten Anzeigenhülse 15 angebracht, sondern helixartig mit in Vorschubrichtung hintereinander und um die Mantelfläche der zweiten Anzeigenhülse umlaufend aufgebrachten Zahlenwerten. Die Zahlenwerte für die Dosiseinheiten können gegenüber dem Ausführungsbeispiel der Figuren 1 bis 4 vergrössert dargestellt und dadurch leichter und sicherer abgelesen werden. Dementsprechend ist die zweite Anzeigenhülse 17 auch nicht am Gehäuse in ihrer axialen Position fixiert. Sie wird vielmehr beim Dosieren und der dazu erfolgenden Drehbewegung des Dosierknopfs 9 am Gehäuse 1 entlang nach vorn verschoben, so dass ein Fenster 31 der zweiten Anzeigenhülse 15 zum Ablesen der Skala diese Skala beim Dosieren überstreicht. Im übrigen kann der Pen der Figur 5 als dem Pen der Figuren 1 bis 4 entsprechend angesehen werden, d.h. etwaige weitere konstruktive Unterschiede sind in Bezug auf die Erfindung ohne Belang. Dementsprechend sind Bauteile gleicher Funktion mit den bereits in den Figuren 1 bis 4 verwendeten Bezugszeichen versehen, und es wird auf die diesbezügliche Beschreibung zu den Figuren 1 bis 4 verwiesen.

Bei diesem Ausführungsbeispiel wird vorzugsweise auf die Einschränkung der Verdrehbarkeit der zweiten Anzeigehülse 17 gegenüber der ersten Anzeigehülse 15 verzichtet.

Die erste Anzeigenhülse 15 bildet beim Pen der Figur 5 gleichzeitig das hintere Gehäuseteil 1, in dem der Antrieb untergebracht ist.

Zum Einstellen der auszuschüttenden Dosis wird der Dosierknopf 9 um seine Längsachse verdreht. Durch die wiederum bei 10 und 20 ausgebildete Verdrehsicherung wird die zweite Anzeigenhülse 17 zwangsweise mitverdreht. Diese zwangsweise Verbindung zwischen Dosierknopf 9 und zweiter Anzeigehülse 17 lässt dem Anwender die Wahl, zur Einstellung der Dosis die zweite Anzeigehülse 17 oder den Dosierknopf 9 zu verdrehen. Die zweite Anzeigenhülse 17 ist mit dem hinteren Gehäuseteil 1 mittels eines Gewindes 33 verbunden. Diese Verbindung bewirkt ein zwangsweises Vorwärtsschieben der zweiten Anzeigenhülse 17 bei einer Verdrehung des Dosierknopfs 9 in dessen ersten Position.

Die Dosiseinstellung "0" ist unmittelbar auf dem Startknopf 14 angebracht, der im übrigen ebenso wie der Nocken 12 und die Feder 13 dem Ausführungsbeispiel der Figuren 1 bis 4 entsprechend ausgebildet ist und mit der Ringnut 11 des Dosierknopfs 9 zusammenwirkt. Die Durchbrechung 19 und das Anzeigenfenster fallen bei der zweiten Anzeigenhülse 17 des Ausführungsbeispiels nach Figur 5 zusammen, d.h. die Dosiswerte der Skala werden im Bereich der Durchbrechung 19 abgelesen, die gleichzeitig wieder das Mittel zur Freigabe der Dosierknopfsicherung bildet. Hierfür ist die Durchbrechung 19 in Form eines U-förmigen Schlitzes ausgebildet. Es ragt in die Durchbrechung 19 eine Lasche 30 hinein, die ganz einfach durch Ausbildung des U-förmigen Schlitzes in der zweiten Anzeigenhülse 17 gebildet sein kann oder, wie im Ausführungsbeispiel, durch eine aus der äusseren Mantelfläche erhaben vorragende Lasche gebildet wird. Die Lasche 30 kann so besonders einfach radial nach innen gedrückt werden. Sie kommt nach dem Loslassen aufgrund ihrer elastischen Ausbildung wieder in ihre Ausgangsposition zurück.

Ein Bereich der elastischen Lasche 30, im Ausführungsbeispiel der vordere Bereich, ist als Fenster 31 ausgebildet. Zur besseren und sicheren Ablesbarkeit der Skala der ersten Anzeigenhülse 15 wirkt das Fenster 31 als Vergrösserungsmittel bzw. Lupe.

Befindet sich der Dosierknopf in seiner vorderen zweiten Position, in der er durch den formschlüssig in die Nut 11 hineinragenden Nocken 12 gehalten wird, wird die zweite Anzeigenhülse 17 der Skala und dem Gewinde 33 folgend zurückgedreht und dabei gleichzeitig nach hinten verschoben, bis der Startknopf 14 mit der aufgedruckten "0" von dem Fenster 31 der Lasche 30 überdeckt wird. In dieser Stellung wird der Startknopf 14 durch Eindrücken der Lasche 30 wie bereits im Ausführungsbeispiel der Figuren 1 bis 4 quer verschoben, so dass der Nocken 12 aus der Nut 11 hinausgedrückt wird und der Dosierknopf 9 mit dem Antrieb 5, 7 in die erste Position zurückfährt.

In einer weiteren Abwandlung des Mittels zum Lösen der Dosierknopfsicherung kann eine Art Lasche auch durch eine durchsichtige, elastische Halbschale gebildet werden, die eine dann wieder einfach flächige Durchbrechung abdeckt und dabei vorzugsweise eine aus der äusseren Mantelfläche der zweiten Anzeigenhülse erhaben vorstehende Eindrückhilfe bildet. Ebenso könnte eine Eindrückhilfe der Lasche 30 vergleichbar, bei der zweiten Anzeigenhülse 17 des Ausführungsbeispiels der Figuren 1 bis 4 verwendet werden.

## Patentansprüche

1. Vorrichtung zur dosierten Verabreichung eines injizierbaren Produkts mit einer Dosierknopfsicherung, wobei die Vorrichtung umfasst:
a) ein Gehäuse (1) mit einer Aufnahme (2) für das Produkt und einem Fördermittel (4) für eine gewählte Dosis,
b) ein Antriebsmittel (5, 7) für das Fördermitel (4),
c) einen mit dem Antriebsmittel (5, 7) gekoppelten, im Gehäuse (1) verschiebbar gelagerten Dosierknopf (9), der zur Auswahl einer Dosis zwischen einer ersten Drehstellung für die Nullstellung und einer zweiten Drehstellung drehbar angeordnet ist, und zum Fördern einer Dosis zwischen einer ersten Position und einer zweiten Position verschiebbar angeordnet ist,
d) eine gehäusefeste erste Anzeigenhülse (15) und eine mit dem Dosierknopf (9) drehbare zweite Anzeigenhülse (17), die Anzeigemittel (16, 18) aufweisen, die ein Ablesen der gewählten Dosis aufgrund der Drehstellung der Anzeigenhülsen (15, 17) zueinander gestatten, und
e) die Dosierknopfsicherung in Form einer Verschiebesicherung (11, 12, 13), die ein Verschieben des Dosierknopfs (9) aus der zweiten Position zurück in die erste Position nur zulässt, wenn die Anzeigenhülsen (15, 17) in einer die Dosis "Null" anzeigenden Nullstellung zueinander stehen,
**dadurch gekennzeichnet, dass**
f) ein Startknopf (14) vorhanden ist, wobei die Verschiebesicherung (11, 12, 13) durch Drücken des Startknopfs (14) gelöst wird und
g) die zweite Anzeigenhülse (17) so ausgebildet ist, dass sie den Startknopf (14) für einen äußeren Zugriff nur in der Nullstellung der Anzeigenhülsen (15, 17) freigibt.

2. Vorrichtung nach Anspruch 1, **dadurch gekennzeichnet, dass** die zweite Anzeigenhülse (17) eine Durchbrechung (19) aufweist, die in der Nullstellung der Anzeigenhülsen (15, 17) radial über dem Startknopf (14) steht.

3. Vorrichtung nach dem vorhergehenden Anspruch, **dadurch gekennzeichnet, dass** die Durchbrechung (19) durch einen an einem stirnseitigen Umfangsrand der zweiten Anzeigenhülse (17) offenen Einschnitt gebildet wird.

4. Vorrichtung nach einem der vorhergehenden Ansprüche, **dadurch gekennzeichnet, dass** mittels der Durchbrechung (19) an der zweiten Anzeigenhülse (17) eine elastische Eindrückhilfe (30) gebildet wird, die in der Nullstellung der Anzeigenhülsen (15, 17) radial über dem Startknopf (14) steht und gegen den Startknopf (14) drückbar ist.

5. Vorrichtung nach einem der vorhergehenden Ansprüche, **dadurch gekennzeichnet, dass** die zweite Anzeigenhülse (17) den Dosierknopf (9) umgebend am Gehäuse (1) um eine Verschiebeachse des Dosierknopfs (9) drehgelagert ist.

6. Vorrichtung nach einem der vorhergehenden Ansprüche, **dadurch gekennzeichnet, dass** die zweite Anzeigenhülse (17) die erste Anzeigenhülse (15) umgibt und die gewählte Dosis durch ein Fenster (18; 31) der zweiten Anzeigenhülse (17) von einer Skala (16) auf der ersten Anzeigenhülse (15) ablesbar ist.

7. Vorrichtung nach einem der vorhergehenden Ansprüche, **dadurch gekennzeichnet, dass** der Dosierknopf (9) und die zweite Anzeigenhülse (17) in der ersten Position des Dosierknopfs (9) mittels einer Verdrehsicherung (10, 20) miteinander gekoppelt sind.

8. Vorrichtung nach Anspruch 7, **dadurch gekennzeichnet, dass** die Kopplung ein Formschluss ist.

9. Vorrichtung nach dem vorhergehenden Anspruch, **dadurch gekennzeichnet, dass** die Verdrehsicherung durch eine zahnartige Paarung von sich axial an einer Mantelfläche des zylindrischen Dosierknopfs (9) erstreckenden Erhebungen und Vertiefungen (10) und damit in Eingriff stehenden, sich axial an einer Mantelfläche der zweiten Anzeigenhülse (17) erstreckenden Erhebungen und Vertiefungen (20) gebildet wird.

10. Vorrichtung nach einem der Ansprüche 7 bis 9, **dadurch gekennzeichnet, dass** die Verdrehsicherung (10, 20) zwischen dem Dosierknopf (9) und der zweiten Anzeigenhülse (17) in der zweiten Position des Dosierknopfs (9) gelöst und die zweite Anzeigenhülse (17) der ersten Anzeigenhülse (15) gegenüber drehbar ist.

11. Vorrichtung nach einem der vorhergehenden Ansprüche, **dadurch gekennzeichnet, dass** zwischen dem Gehäuse (1) und der zweiten Anzeigenhülse (17) ein in einer Drehrichtung der zweiten Anzeigenhülse (17) wirkender Anschlag (22, 23) gebildet ist, der die maximal wählbare Dosis begrenzt.

12. Vorrichtung nach einem der vorhergehenden Ansprüche, **dadurch gekennzeichnet, dass** die Vorrichtung als Injektionspen verwendet wird.

## Claims

1. A device for administering an injectable product in doses, comprising a system for locking a dosing button, said apparatus comprising:
a) a housing (1), including an accommodation (2) for said product and a delivery member (4) for a selected dose;
b) a drive member (5, 7) for said delivery member (4);
c) a dosing button (9) which is coupled to said drive member (5, 7), shiftably mounted in said housing (1), and arranged such that it can be rotated between a first rotation position for zero delivery and a second rotation position, in order to select a dosage, and shifted between a first position and a second position in order to deliver a dosage;
d) a first indicator sleeve (15), affixed to said housing, and a second indicator sleeve (17), rotatable with said dosing button (9), said indicator sleeves comprising indicator members (16, 18) which allow said selected dose to be read, based on the rotation position of said indicator sleeves (15, 17) relative to each other; and
e) the system for locking a dosing button, in the form of an anti-shifting member (11, 12, 13) which only allows said dosing button (9) to be shifted from said second position back to said first position when said indicator sleeves (15, 17) are located in a zero position, indicating a "zero" dose, relative to each other;
**characterized in that**
f) a trigger button (14) is provided, wherein said anti-shifting member (11, 12, 13) is released by pressing said trigger button (14); and
g) said second indicator sleeve (17) is formed such that it only releases said trigger button (14) for external access in said zero position of said indicator sleeves (15, 17).

2. The device as set forth in claim 1, **characterized in that** said second indicator sleeve (17) comprises a breakthrough (19) which is located radially above said trigger button (14) in said zero position of said indicator sleeves (15, 17).

3. The device as set forth in the preceding claim, **characterized in that** said breakthrough (19) is formed by a recess which is open on a facing circumferential edge of said second indicator sleeve (17).

4. The device as set forth in any one of the preceding claims, **characterized in that** an elastic pressing aid (30) is formed by means of said breakthrough (19) on said second indicator sleeve (17), wherein in said zero position of said indicator sleeves (15, 17), said pressing aid (30) protrudes radially above said trigger button (14) and can be pressed against said trigger button (14).

5. The device as set forth in any one of the preceding claims, **characterized in that** said second indicator sleeve (17) is rotatively mounted on said housing (1) about a shifting axis of said dosing button (9), surrounding said dosing button (9).

6. The device as set forth in any one of the preceding claims, **characterized in that** said second indicator sleeve (17) surrounds said first indicator sleeve (15), and said selected dose can be read through a window (18; 31) of said second indicator sleeve (17) from a scale (16) on said first indicator sleeve (15).

7. The device as set forth in any one of the preceding claims, **characterized in that** in said first position of said dosing button (9), said dosing button (9) and said second indicator sleeve (17) are coupled to each other by means of an anti-rotation lock (10, 20).

8. The device as set forth in claim 7, **characterized in that** the coupling is a positive lock.

9. The device as set forth in the preceding claim, **characterized in that** said anti-rotation lock is formed by a meshing pairing of ridges and furrows (10) extending axially over a shell surface of said cylindrical dosing button (9) and meshing ridges and furrows (20) extending axially over a shell surface of said second indicator sleeve (17).

10. The device as set forth in any one of claims 7 to 9, **characterized in that** in said second position of said dosing button (9), said anti-rotation lock (10, 20) between said dosing button (9) and said second indicator sleeve (17) is released and said second indicator sleeve (17) can be rotated relative to said first indicator sleeve (15).

11. The device as set forth in any one of the preceding claims, **characterized in that** a stop (22, 23) is formed between said housing (1) and said second indicator sleeve (17), said stop (22, 23) acting in one direction of rotation of said second indicator sleeve (17), to define a maximally selectable dose.

12. The device as set forth in any one of the preceding claims, **characterized in that** said device is used as an injection pen.

## Revendications

1. Dispositif servant à administrer de manière dosée un produit injectable avec un système de blocage pour le bouton de dosage, dans lequel le dispositif comprend :
a) un boîtier (1) avec un réceptacle (2) pour le produit et avec un moyen de transport (4) pour une dose sélectionnée,
b) un moyen d'entraînement (5, 7) pour le moyen de transport (4),
c) un bouton de dosage (9) couplé avec le moyen d'entraînement (5, 7) et logé de manière déplaçable dans le boîtier (1) qui, pour la sélection d'une dose, est disposé de manière tournante entre une première position de rotation pour la position zéro et une deuxième position de rotation, et qui, pour transporter une dose, est disposé de manière déplaçable entre une première position et une deuxième position,
d) une première douille indicatrice (15) solidaire du boîtier et une deuxième douille indicatrice (17) pouvant être tournée avec le bouton de dosage (9) comprenant des moyens indicateurs (16, 18) qui, sur la base de la position de rotation des douilles indicatrices (15, 17) entre elles, permettant une lecture de la dose sélectionnée et
e) le système de blocage pour le bouton de dosage sous la forme d'une sécurité anti-déplacement (11, 12, 13) qui ne permet un déplacement du bouton de dosage (9) à partir de la deuxième position dans le sens du retour dans la première position que lorsque les douilles indicatrices (15, 17) sont placées l'une par rapport à l'autre dans une position zéro indiquant la dose "zéro",
caractérisé
f) en ce qu'il existe un bouton de démarrage (14), la sécurité anti-déplacement (11, 12, 13) étant déclenchée par une poussée sur le bouton de démarrage (14) et
g) en ce que la deuxième douille indicatrice (17) est configurée de telle façon qu'elle ne libère le bouton de démarrage (14) pour un accès extérieur que dans la position zéro des douilles indicatrices (15, 17).

2. Dispositif selon la revendication 1, **caractérisé en ce que** la deuxième douille indicatrice (17) comprend une traversée (19) qui, dans la position zéro des douilles indicatrices (15, 17), se trouve en position radiale au-dessus du bouton de démarrage (14).

3. Dispositif selon la revendication précédente, **caractérisé en ce que** la traversée (19) est formée par une entaille ouverte sur un bord périphérique frontal de la deuxième douille indicatrice (17).

4. Dispositif selon l'une quelconque des revendications précédentes, **caractérisé en ce qu'**à l'aide de la traversée (19) est formé, sur la deuxième douille indicatrice (17), un poussoir souple (30) qui, dans la position zéro des douilles indicatrices (15, 17), se trouve en position radiale au-dessus du bouton de démarrage (14) et peut être poussé contre le bouton de démarrage (14).

5. Dispositif selon l'une quelconque des revendications précédentes, **caractérisé en ce que** la deuxième douille indicatrice (17) est, tout en entourant le bouton de dosage (9), montée rotative autour d'un axe de déplacement du bouton de dosage (9) sur le boîtier (1).

6. Dispositif selon l'une quelconque des revendications précédentes, **caractérisé en ce que** la deuxième douille indicatrice (17) entoure la première douille indicatrice (15) et que la dose choisie peut être lue sur la première douille indicatrice (15) à travers une fenêtre (18 ; 31) de la deuxième douille indicatrice (17) sur une échelle graduée (16).

7. Dispositif selon l'une quelconque des revendications précédentes, **caractérisé en ce que**, dans la première position du bouton de dosage (9), le bouton de dosage (9) et la deuxième douille indicatrice (17) sont couplés ensemble à l'aide d'une sécurité anti-rotation (10, 20).

8. Dispositif selon la revendication 7, **caractérisé en ce que** le couplage est une liaison en complémentarité de forme.

9. Dispositif selon la revendication précédente, **caractérisé en ce que** la sécurité anti-rotation est formée par un appariement en forme de dents de saillies et de creux (10) s'étendant dans le sens axial sur une surface d'enveloppe du bouton de dosage cylindrique (9) et de saillies et de creux (20) en prise avec ceux-ci et s'étendant dans le sens axial sur une surface d'enveloppe de la deuxième douille indicatrice (17).

10. Dispositif selon l'une quelconque des revendications 7 à 9, **caractérisé en ce que**, dans la deuxième position du bouton de dosage (9), la sécurité anti-rotation (10, 20) entre le bouton de dosage (9) et la deuxième douille indicatrice (17) est dégagée et **en ce que** la deuxième douille indicatrice (17) peut être tournée par rapport à la première douille indicatrice (15).

11. Dispositif selon l'une quelconque des revendications précédentes, **caractérisé en ce qu'**entre le boîtier (1) et la deuxième douille indicatrice (17) est formée une butée (22, 23) agissant dans un sens de rotation de la deuxième douille indicatrice (17) et qui limite la dose maximale pouvant être sélectionnée.

12. Dispositif selon l'une quelconque des revendications précédentes, **caractérisé en ce que** le dispositif est utilisé comme stylo d'injection.
